# EUROPEAN PATENT APPLICATION

(11) **EP 4 610 246 A1**
(43) Date of publication of application: **03.09.2025**
(21) Application number: 23881909.8
(22) Date of filing: 26.10.2023
(51) Int. Cl.: C07C 27/00, B01J 31/16, B01J 31/22, C07C 31/10, C07C 35/06, C07C 35/08

(54) **METAL OXYGEN-CONTAINING COMPLEX AND USE THEREOF AS PRE-OXIDANT**

(30) Priority: 26.10.2022 CN 202211316426
(71) Applicant: China Petroleum & Chemical Corporation, Beijing 100728 (CN); Sinopec (Shangai) Research Institute of Petrochemical Technology Co., Ltd., Shanghai 201208 (CN)
(72) Inventor: ZONG, Hongyuan, Shanghai 201208 (CN); YU, Qiang, Shanghai 201208 (CN); LIU, Xu, Shanghai 201208 (CN); WANG, Yanhong, Shanghai 201208 (CN); CAO, Jun, Shanghai 201208 (CN); CHEN, Liang, Shanghai 201208 (CN); LIU, Xiaoxi, Shanghai 201208 (CN); QIN, Lei, Shanghai 201208 (CN); BAI, Xue, Shanghai 201208 (CN)
(74) Representative: karo IP
(86) International application number: PCT/CN2023/126697
(87) International publication number: WO 2024/088333

(57) **Abstract**

The present invention provides a metal oxygen-containing complex, which comprises a transition metal element as central atom and an oxygen-containing heterocyclic organic compound containing a substituent as ligand; the ligand is a compound having a structure shown in formula (I).

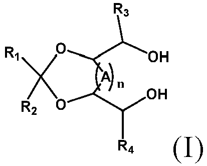

## Description

### Technical Field

The invention relates to the technical field of chemical raw material preparation, and in particular to a metal oxygen-containing complex and a use thereof as a pre-oxidant.

### Background Art

With the acceleration of the third industrial transfer of the semiconductor industry since the 21st century, the scale of China's integrated circuit market is growing year by year. Ultrahigh-purity reagents (wet electronic chemicals) are key basic functional chemicals in the IC manufacturing process, mainly used for chip cleaning and etching. Their purity and cleanliness have a very important impact on the yield and reliability of integrated circuits, and the narrower the integrated circuit line width is, the higher the required quality requirement is. At present, 8-inch wafer production uses G3-G4 grade wet electronic chemicals. Due to the change in processing mode of 12-inch wafers, they have greatly increased the use of wet electronic chemicals and put forward higher requirements for the grade of wet electronic chemicals, generally requiring G4-G5 grades.

Isopropanol is the most widely used organic solvent in wet electronic chemicals, mainly used for cleaning and drying. isopropanol for integrated circuits has strict requirements on trace organic impurities content, metal cation impurities content, particle size and number, anion impurities content, etc. With the continuous breakthroughs in advanced process nodes of integrated circuits, the demand for G4-G5 high-grade isopropanol has gradually increased.

CN111675598A discloses a production system for preparing electronic-grade isopropanol by hydrogenation of acetone, wherein the electronic-grade isopropanol product is obtained through an acetone hydrogenation unit, a variable pressure adsorption unit, a distillation unit and an adsorption-filtration unit. However, this process adopts the variable pressure adsorption separation process which makes the process complicated, and the measured trace organic impurities in the obtained isopropanol product are not effectively removed, which cannot meet the demands of high-end wafer manufacturing (line width≤90nm).

CN102898275A discloses a process for preparing highly-pure isopropanol, wherein highly-pure isopropanol is prepared by molecular sieve dehydration, resin dehydration, reverse osmosis, high-temperature distillation, ion exchange, and cyclic adsorption-filtration. However, this process also does not consider the removal of trace organic impurities and cannot meet the cleanliness requirements of wet electronic chemicals for high-end wafer manufacturing (line width≤90nm).

### Summary of the Invention

One of the objects of the present invention is thus, in order to overcome the problem that the reducing oxygen-containing organic impurities in the isopropanol obtained from the production are not effectively removed and cannot meet the requirements of high-end wafer fabrication (line width≤90nm), to provide a process and system for producing electronic-grade isopropanol by hydrogenation of acetone. The organic impurities content in the isopropanol product obtained by the process can be as low as below 5ppm, and the anions and cations, particle content, water content, etc. can all meet the requirements of high-end wafer manufacturing (line width≤90nm).

To this end, the inventors has conducted extensive research. During the research, the inventors unexpectedly found that for some specific crude material including acetone crude material for the production of electronic-grade isopropanol, including ketones crude material and monocyclic aromatic crude material, especially when they contain specific impurities, such as reducing impurities, specific metal oxygen-containing complexes can be used as pre-oxidants to pre-treat the crude material, which is particularly beneficial to reduce the content of organic impurities in the desired product and meet the multifaceted requirements of anions and cations, particle content, water content, etc.

In order to achieve the above object, in one aspect, the present invention provides a metal oxygen-containing complex, which comprises a transition metal element as central atom and an oxygen-containing heterocyclic organic compound containing a substituent as ligand;
wherein the ligand is a compound having a structure shown in formula (I), 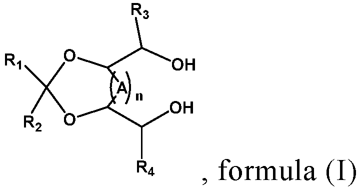
in formula (I), n is an integer of 0-5, A is -CH(R₅)-;
R₁ and R₂ at each occurrence are each independently selected from C1-C8 alkyl, and R₃, R₄ and R₅ at each occurrence are each independently selected from H, C1-C8 alkyl, phenyl C1-C8 alkyl and C1-C8 alkylphenyl.

In another aspect, the present invention provides a process for treating a crude material by using the metal oxygen-containing complex of the present invention, which comprises using the metal oxygen-containing complex as a pre-oxidant to be in contact with the crude material, thereby pre-oxidizing at least part of the impurities contained in the crude material, wherein the impurities include reducing impurities.

In another aspect, the present invention provides a process for preparing electronic-grade isopropanol by hydrogenating acetone, the process comprising:
(1) introducing the acetone raw material into an acetone pre-oxidation processor to pre-oxidize impurities to obtain pre-oxidized acetone;
(1-2) introducing the pre-oxidized acetone raw material into an acetone dehydration device and an acetone refining device in sequence to perform acetone dehydration and acetone refining to obtain refined acetone;
(2) introducing the refined acetone into an acetone hydrogenation reactor for hydrogenation reaction to obtain a crude isopropanol product;
(3) passing the crude isopropanol product sequentially through an isopropanol dehydration device, an isopropanol refining device, and an adsorption-filtration device to perform isopropanol dehydration, isopropanol refining and at least one isopropanol adsorption-filtration, to obtain electronic-grade isopropanol.

In another aspect, the present invention provides a system for preparing electronic-grade isopropanol by hydrogenating acetone, the system comprising:
an acetone purification unit, which is used to purify the acetone raw material to obtain acetone feed;
an acetone hydrogenation unit, which is used to hydrogenate the acetone feed to obtain a crude isopropanol product;
an isopropanol purification unit, which is used to purify the crude isopropanol product to obtain electronic-grade isopropanol;
wherein, the acetone purification unit comprises an acetone pre-oxidation processor, an acetone dehydration device, and an acetone refining device which are connected in sequence; the acetone hydrogenation unit comprises an acetone hydrogenation reactor; and the isopropanol purification unit comprises an isopropanol dehydration device, an isopropanol refining device, and an adsorption-filtration device which are connected in sequence.

The present invention provides a metal oxygen-containing complex, which can be used as a pre-oxidant to treat a crude material selected from a ketones crude material and a monocyclic aromatic hydrocarbons crude material, especially used for a process for hydrogenating acetone to produce electronic-grade isopropanol. Compared with the prior art, the metal oxygen-containing complex provided by the present invention has at least the following beneficial effects when it is used for hydrogenating acetone to produce electronic-grade isopropanol:
(1) the content of organic reducing impurities in the isopropanol product obtained by the present invention can be as low as below 5 ppm, and the anion and cation, particle content, water content, etc. can all meet the requirements of high-end wafer manufacturing (line width≤90 nm);
(2) the raw material used in the present invention can be cheap and easily available acetone, and an electronic-grade isopropanol product is obtained through an acetone purification unit, an acetone hydrogenation unit and an isopropanol purification unit. The overall process is simple and the process added value is high;
(3) the acetone purification unit used in the present invention has mild reaction conditions and a good oxidation effect. It can completely remove reducing impurities with almost no loss of raw material acetone and has few by-products;
(4) the present invention removes the organic impurities, which are difficult to be separated from isopropanol, in the acetone purification unit, thereby avoiding the difficulty of separating from isopropanol and high energy consumption in the later stage.

### Brief Description of the Figures

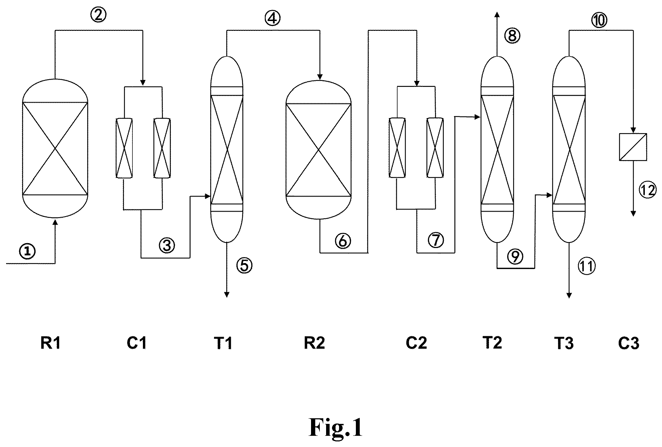

FIG.1 is a system for producing electronic-grade isopropanol by hydrogenating acetone according to a preferred embodiment of the present invention.

### Description of Reference Numerals

| | | | |
|---|---|---|---|
| R1: | Acetone pre-oxidation processor | C1: | Acetone dehydration tower |
| T1: | Acetone refining tower | R2: | Acetone hydrogenation reactor |
| C2: | Isopropanol dehydration tower | T2: | Isopropanol de-light tower |
| T3: | Isopropanol de-heavy tower | C3: | Adsorption-filtration equipment |
| ① | Acetone raw material | ② | Pre-oxidized acetone |
| ③ | Dehydrated acetone | ④ | Refined acetone feed |
| ⑤ | Heavy components | ⑥ | Crude isopropanol product |
| ⑦ | Dehydrated isopropanol | ⑧ | Light impurity components |
| ⑨ | De-light isopropanol products | ⑩ | Primary isopropanol products |
| ⑪ | Heavy impurity components | ⑫ | Electronic-grade isopropanol |

### Detailed Description of The Invention

The endpoints and any values of the ranges disclosed herein are not limited to the precise ranges or values, and these ranges or values should be understood to include values close to these ranges or values. For numerical ranges, the endpoint values of each range, the endpoint values of each range and the individual point values, and the individual point values can be combined with each other to obtain one or more new numerical ranges, which should be considered as specifically disclosed herein.

The "crude material" of the present invention such as ketones crude material (such as acetone crude material) and monocyclic aromatic crude material, contains target material (e.g., the target material in the acetone crude material is acetone) and impurities. For the object of further processing or application, it is necessary to perform appropriate treatment, such as purification, so as to improve the purity of the target material and/or facilitate the further processing or application of the target material. For the present invention, the crude material in particular may refer to the acetone raw material used for hydrogenation to produce isopropanol (especially electronic-grade isopropanol).

In one aspect, the present invention provides a metal oxygen-containing complex comprising a transition metal element as central atom and an oxygen-containing heterocyclic organic compound containing a substituent as ligand;
the ligand is a compound having a structure shown in formula (I), 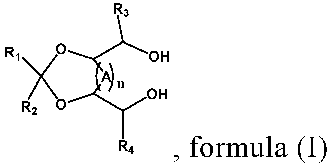
in formula (I), n is an integer of 0-5, A is -CH(R₅)-;
R₁ and R₂ at each occurrence are each independently selected from C1-C8 alkyl, and R₃, R₄ and R₅ at each occurrence are each independently selected from H, C1-C8 alkyl, phenyl C1-C8 alkyl and C1-C8 alkylphenyl.

According to a preferred embodiment of the present invention, R₁ and R₂ at each occurrence are each independently selected from C1-C5 alkyl, preferably each independently selected from C1-C4 alkyl; R₃, R₄ and R₅ are each independently selected from H, C1-C5 alkyl, phenyl C1-C5 alkyl and C1-C5 alkyl-substituted phenyl, preferably each independently selected from H, C1-C4 alkyl, benzyl and C1-C3 alkyl-substituted phenyl.

According to a preferred embodiment of the present invention, in formula (I), n is an integer of 0-3; R₁ and R₂ are each independently selected from H, C1-C3 alkyl, R₃ and R₄ are each independently selected from H, C1-C3 alkyl, benzyl and methyl-substituted phenyl; and R₅ is H or C1-C3 alkyl. Preferably, in formula (I), n is 0 or 1; and R₁ and R₂ are each independently selected from C1-C3 alkyl, R₃ and R₄ are each independently selected from H, C1-C3 alkyl, benzyl and methyl-substituted phenyl, and R₅ is H.

According to the present invention, as long as the object of the present invention can be achieved, there is no limitation on the type of transition metal element of the metal oxygen-containing complex. In some embodiments, the transition metal element is selected from one or more of chromium, molybdenum, tungsten, iron, cobalt, nickel, ruthenium, rhodium, palladium, platinum, manganese, technetium and rhenium, preferably one or more of manganese, cobalt and molybdenum.

According to the present invention, it can be understood that when n is 0, then there is no A, and then the formula (I) is a five-membered ring structure; when n is 1, then the formula (I) is a six-membered ring structure, and then R₅ is selected from H, C1-C8 alkyl, phenyl C1-C8 alkyl and C1-C8 alkylphenyl, or preferably selected from H, C1-C5 alkyl, benzyl, C1-C3 alkyl-substituted phenyl; when n is 2, then A=-CH(R₅)-CH(R₅), the formula (I) is a seven-membered ring structure, and R₅ at each occurrence is independently selected from H, C1-C8 alkyl, phenyl C1-C8 alkyl and C1-C8 alkylphenyl, or preferably selected from H, C1-C5 alkyl, benzyl, C1-C3 alkyl-substituted phenyl. Similarly, for example:
when n is 0, R₁ is methyl, R₂ is methyl, R₃ is H, and R₄ is H, then the ligand is 2,2-dimethyl-1,3-dioxolane-4,5-dimethanol;
when n is 0, R₁ is ethyl, R₂ is ethyl, R₃ is H, and R₄ is H, then the ligand is 2,2-diethyl-1,3-dioxolane-4,5-dimethanol.

Similarly, compounds of formula (I) useful in the present invention also include 2,2-dimethyl-1,3-dioxolane-4,5-di-1-ethanol, 2,2-dimethyl-1,3-dioxolane-4,5-dibenzyl alcohol, and 2,2-dimethyl-1,3-dioxolane-4,5-di-p-methylbenzyl alcohol.

According to the present invention, as long as the object of the present invention can be achieved, there is no limitation on the preparation process of the metal oxygen-containing complex, and it can be obtained by selecting a ligand solution containing the ligand of the present invention and a metal salt solution containing the transition metal of the present invention to conduct a complexation reaction as needed. There is no limitation on the conditions required for the complexation reaction and they can be selected as needed. In some embodiments, the preparation process of the metal oxygen-containing complex comprises: adding a ligand solution and a metal salt solution in an anhydrous, oxygen-free, nitrogen atmosphere according to equimolar ligand and transition metal, stirring at room temperature for 1-10 hours to conduct a complexation reaction; in some optional embodiments, the solvent is evaporated after the complexation reaction.

According to the present invention, it can be understood that the ligand solution refers to a solution formed by dissolving the ligand in a solvent, generally it is dissolved in an organic solvent; the metal salt solution refers to a solution formed by dissolving a metal salt in a solvent, generally it is dissolved in water. The present invention has no special limitations on this and it will not be elaborated herein.

According to the present invention, the ligand can be obtained by preparation as needed, for example, when in formula (I), n is 0, R₁ is methyl, R₂ is methyl, R₃ is H, and R₄ is H, then the ligand is 2,2-dimethyl-1,3-dioxolane-4,5-dimethanol. The preparation process of the ligand comprises: sequentially adding 2,2-dimethoxypropane, dimethyl 2,3-dihydroxysuccinate, p-toluenesulfonic acid and anhydrous dichloromethane in an anhydrous and oxygen-free nitrogen atmosphere, performing a reaction at 20-100°C for 2-10 hours, and evaporating the solvent after natural cooling; adding anhydrous methanol and sodium borohydride in a nitrogen atmosphere, and stirring at room temperature for 5-24 hours; diluting with water and then evaporating the organic solvent, extracting the residual liquid and then drying it, filtering and evaporating the organic solvent, and recrystallizing the residue to obtain the ligand.

The present invention does not elaborate on the source of the ligand. The ligand in the present invention can be prepared by a similar preparation process to 2,2-dimethyl-1,3-dioxolane-4,5-dimethanol, comprising: changing the raw materials, 2-dimethoxypropane, dimethyl 2,3-dihydroxysuccinate, selecting the raw materials for preparing the required ligand, reacting them under the action of p-toluenesulfonic acid, and then performing a reduction reaction.

The metal oxygen-containing complex of the present invention has a certain oxidizing property and is particularly suitable as a pre-oxidant. Therefore, in another aspect, the present invention provides the use of the metal oxygen-containing complex of the present invention as a pre-oxidant to pre-oxidize an object to be oxidized. Without being limited to any known theory, according to the present invention, due to the redox properties of the metal oxygen-containing complex of the present invention, the object to be oxidized particularly may be, for example, ketones and monocyclic aromatic compounds.

Correspondingly, in another aspect, the present invention provides a process for treating a crude material selected from ketones crude material and monocyclic aromatic hydrocarbons crude material by using the metal oxygen-containing complex of the present invention, the process comprising using the metal oxygen-containing complex of the present invention as a pre-oxidant to contact the crude material, thereby pre-oxidizing at least part of the impurities contained in the crude material, wherein the impurities include reducing impurities.

According to the present invention, as long as the object of the present invention can be achieved, there is no limitation on the pre-oxidation method. In some preferred embodiments, in step (1), the pre-oxidation is carried out by contacting the crude material with a pre-oxidant. By adopting the above-mentioned embodiment, the reducing impurities in the crude material can be effectively removed.

According to the present invention, as long as the object of the present invention can be achieved, there is no limitation on the pre-oxidation conditions. In some embodiments, in step (1), the pre-oxidation conditions include: the amount of the pre-oxidant added is 0.01-0.1% by weight of the crude material, such as 0.01%, 0.02 %, 0.03 %, 0.04 %, 0.05%, 0.08% or 0.1%. By adopting the above embodiment, the reducing impurities in the crude material can be removed.

According to the present invention, a crude material selected from ketones crude material and monocyclic aromatic hydrocarbons crude material can be treated by using the metal oxygen-containing complexes described in the present invention. Without being limited to any known theory, it is believed that ketones containing C1-C8 alkanes, C6-C8 aromatic rings, C2-C8 ester groups, halogens, or monocyclic aromatic hydrocarbons containing C1-C8 alkanes, C2-C8 ester groups, halogens are particularly suitable for being treated by the process of the present invention. The halogen can be selected from F, Cl, Br and I, preferably selected from F and Cl.

In various cases, especially for different crude materials, the process of the present invention can be applied to a variety of different reducing impurities, such as alcohols, aldehydes, ketones, esters, ethers and olefins. Generally speaking, when the crude material treated by the process of the present invention is a ketones crude material or a monocyclic aromatic crude material, the reducing impurities that can be pre-oxidized by the process of the present invention are selected from saturated or unsaturated C1-C4 primary or secondary alcohols, such as methanol, ethanol, isopropanol, propenol, propargyl alcohol; C1-C4 aldehydes, such as formaldehyde, acetaldehyde, propionaldehyde; C1-C4 alkyl formate, such as methyl formate, ethyl formate; C2-C4 olefins or alkynes, such as ethylene, propylene; aniline optionally substituted with C1-C4 alkyl and phenol optionally substituted with C1-C4 alkyl, such as aniline, phenol and alkyl-substituted compounds thereof.

According to the present invention, in some embodiments, the pre-oxidation conditions include: normal temperature and normal pressure. By adopting the above-mentioned embodiments, the pre-oxidation has mild reaction conditions and a good oxidation effect, and the reducing impurities can be completely removed with almost no loss of the crude material, and there are few by-products.

In the present invention, the normal temperature refers to a temperature of 10-30°C.

According to the present invention, in order to increase the activity of the metal oxygen-containing complex, in some embodiments, before using the pre-oxidant to perform pre-oxidation, the pre-oxidant is subjected to an oxidation treatment using an oxidizing agent.

According to the present invention, in some preferred embodiments, the oxidant is selected from one or more of oxygen, hydrogen peroxide, cumene peroxide, peracetic acid, perchlorate, permanganate and dichromate.

According to the present invention, the pre-oxidant can be oxidized by directly adding the oxidant to conduct the oxidation reaction after the complexation reaction for preparing the metal oxygen-containing complex is completed. For example, in some embodiments, according to equimolar amounts of ligand and transition metal, a ligand solution and a metal salt solution are added in an anhydrous and oxygen-free nitrogen atmosphere and stirred at room temperature for 1-10 hours to conduct a complexation reaction, and then an appropriate amount of oxidant is added hereinto and stirred for 1-10 hours to conduct an oxidation treatment, and then the solvent is evaporated to obtain an oxidized pre-oxidant.

According to the present invention, in other embodiments, the pre-oxidant can be oxidized by subjecting the metal oxygen-containing complex to an oxidation reaction with an oxidant under oxidizing conditions in the presence of a solvent, and then evaporating the solvent to obtain an oxidized pre-oxidant.

According to the present invention, those skilled in the art can select appropriate oxidation treatment conditions according to the type of oxidant, and the present invention does not dwell on this too much.

As shown in FIG. 1, in another aspect, the present invention provides a process for preparing electronic-grade isopropanol by hydrogenating acetone, the process comprising:
(1) introducing an acetone raw material into an acetone pre-oxidation processor R1, and using the pre-oxidant of the present invention to pre-oxidize impurities to obtain pre-oxidized acetone;
(1-2) introducing the pre-oxidized acetone into an acetone dehydration device and an acetone refining device in sequence to dehydrate the acetone and refine the acetone to obtain refined acetone;
(2) introducing the refined acetone liquid into the acetone hydrogenation reactor R2 for hydrogenation reaction to obtain a crude isopropanol product;
(3) passing the crude isopropanol product sequentially through an isopropanol dehydration device, an isopropanol refining device, and an adsorption-filtration device to perform isopropanol dehydration, isopropanol refining and at least one isopropanol adsorption-filtration, to obtain electronic-grade isopropanol.

According to the present invention, it is possible to obtain refined acetone by pre-oxidation of cheap and easily available acetone raw material, dehydration of acetone and refining of acetone, and use the refined acetone to conduct a hydrogenation reaction to obtain crude isopropanol products, and the subsequent crude isopropanol product only need simple dehydration, refining and at least one adsorption-filtration to obtain an isopropanol product, of which the organic reducing impurities content is as low as 5ppm or less, and of which the anions and cations, particle content, water content, etc. can all meet the requirements of high-end wafer manufacturing (line width ≤ 90nm). In the present invention, the organic impurities that are difficult to be separated from isopropanol are removed in the acetone raw material, thereby avoiding the defects of difficulty in separating from isopropanol and high energy consumption in the later stage.

According to the present invention, as long as the object of the present invention can be achieved, there is no limitation on the pre-oxidation method. In some preferred embodiments, in step (1), the pre-oxidation is carried out by contacting the acetone raw material with a pre-oxidant. By adopting the above-mentioned embodiments, the impurities in the acetone can be effectively removed.

According to the present invention, as long as the object of the present invention can be achieved, there is no limitation on the pre-oxidation conditions. In some embodiments, in step (1), the pre-oxidation conditions include: the amount of the pre-oxidant added is 0.01-0.1% by weight of the acetone raw material, such as 0.01%, 0.02 %, 0.03%, 0.04%, 0.05%, 0.08% or 0.1%. By adopting the above embodiments, the reducing impurities in the acetone raw material can be removed.

According to the present invention, in some embodiments, the pre-oxidation conditions include: normal temperature and normal pressure. By adopting the above-mentioned embodiment, the reaction conditions of pre-oxidation are mild, the oxidation effect is good, and the reducing impurities can be completely removed with almost no loss of raw material acetone, and there are few by-products.

In the present invention, the normal temperature refers to a temperature of 10-30°C.

The inventors unexpectedly found that, in particular, when used for hydrogenation of acetone to isopropanol, the ligand that can be used as a pre-oxidant, in addition to the compound of formula (I) of the present invention, can also be a compound having a structure shown in formula (I-1), 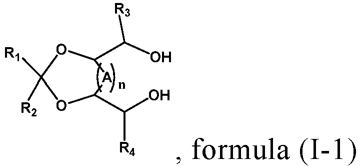 in formula (I), n is an integer from 0 to 5 (e.g., 0, 1, 2, 3, 4 or 5), A is - CH(R₅)-; R₁, R₂, R₃, R₄, R₅ at each occurrence are each independently selected from H, C1-C5 alkyl, benzyl, C1-C3 alkyl-substituted phenyl. It is understood that, for the purpose of the present invention, the compounds of formula (I) and (I-1) have the same main structure, but the definitions of the groups R₁-R₅ are different.

By adopting the above-mentioned preferred embodiments, the organic impurities that are difficult to be separated from isopropanol can be removed in the acetone raw material, thereby avoiding the defects of difficulty in separating from isopropanol and high energy consumption in the later stage.

According to the present invention, as long as the object of the present invention can be achieved, there is no limitation on the type of the reducing impurities. In some embodiments, the reducing impurities include at least one of alcohol, aldehyde, ketone, ester, ether and olefin. The pre-oxidation of the present invention can efficiently remove the reducing impurities such as alcohol, aldehyde, ketone, ester, ether and olefin, and finally produce isopropanol that can meet the requirements of high-end wafer manufacturing (line width ≤ 90nm) in terms of anions and cations, particle content, water content, etc. In general, the reducing impurities that can be treated by the process of the present invention are selected from saturated or unsaturated C1-C4 primary or secondary alcohols, such as methanol, ethanol, isopropanol, propenol, propargyl alcohol; C1-C4 aldehydes, such as formaldehyde, acetaldehyde, propionaldehyde; C1-C4 alkyl formate, such as methyl formate, ethyl formate; C2-C4 olefins or alkynes, such as ethylene and propylene; aniline optionally substituted with C1-C4 alkyl and phenol optionally substituted with C1-C4 alkyl, such as aniline, phenol and alkyl-substituted compound thereof.

According to the present invention, as long as the object of the present invention can be achieved, there is no limitation on the source of the acetone raw material. In some embodiments, the acetone raw material includes one or more of industrial-grade acetone, analytical-grade acetone and electronic-grade acetone.

According to the present invention, according to the standards in this field, the purity of industrial-grade acetone is 95-99.5%, the purity of analytical-grade acetone is 99.5-99.9%, and the purity of electronic-grade acetone is above 99.9%.

In the present invention, unless otherwise specified, purity and content refer to the purity and content by weight.

According to the present invention, in some embodiments, in the dehydration of acetone in step (1-2) and the dehydration of isopropanol in step (3), the dehydrating materials used each include one or more of molecular sieves, silica gel, membranes, resins, and salts capable of forming crystalline hydrates. By adopting the above-mentioned embodiment, the water content of the finally obtained isopropanol product can meet the requirements of ultra-high purity isopropanol used in the integrated circuit industry.

According to the present invention, the dehydrating material can be selected as needed, and the types of the dehydrating material will not be described in detail herein.

According to the present invention, in some embodiments, in step (1-2), the acetone is refined in a plate distillation tower or a packed distillation tower.

According to the present invention, as long as the object of the present invention can be achieved, there is no limitation on the refining conditions of acetone. In some embodiments, the refining conditions of acetone include: an operating reflux ratio of 0.5-10; in some embodiments, the refining conditions of acetone include: an operating pressure of -0.5~10bar; in some embodiments, the refining conditions of acetone include: a tower top operating temperature of 30-70°C.

According to the present invention, in some preferred embodiments, the refining conditions of acetone include: an operating reflux ratio of 0.5-5; in some preferred embodiments, the refining conditions of acetone include: an operating pressure of 0.5-1 bar; in some preferred embodiments, the refining conditions of acetone include: a tower top operating temperature of 35-65°C. By adopting the above embodiments, some impurities with high boiling point in acetone can be removed.

According to the present invention, as long as the object of the present invention can be achieved, there is no limitation on the mode and conditions of the hydrogenation reaction. In some embodiments, the hydrogenation catalyst used in the hydrogenation reaction includes one or more of Cu-based, Ni-based and Mn-based catalysts.

According to the present invention, "Cu-based" refers to a hydrogenation catalyst in which Cu is loaded onto a carrier as an active component; "Mn-based" refers to a hydrogenation catalyst in which Mn is loaded onto a carrier as an active component; the type of carrier can be selected as needed, for example including but not limited to SiO₂ carrier, molecular sieve carrier or Al₂O₃ carrier.

According to the present invention, as long as the object of the present invention can be achieved, there is no limitation on the conditions of the hydrogenation reaction. In some embodiments, the conditions of the hydrogenation reaction include: a reaction inlet temperature of 70-140°C (e.g., 70°C, 80°C, 90°C, 110°C, 120°C, 130°C or 140°C); in some embodiments, the conditions of the hydrogenation reaction include: a reaction pressure of 2-8MPaG (e.g., 2MPaG, 4MPaG, 5MPaG, 7MPaG or 8MPaG); in some embodiments, the conditions of the hydrogenation reaction include: a molar ratio of hydrogen to acetone of 4-40 : 1 (e.g., 4:1, 8:1, 12:1, 16:1, 20:1, 21:1, 23:1, 30:1, 32:1, 35:1 or 40:1). In some embodiments, the conditions of the hydrogenation reaction include: an acetone space velocity of 0.6-3h⁻¹ (e.g., 0.6 h⁻¹, 1h⁻¹, 1.6h⁻¹, 2h⁻¹, 2.5h⁻¹ or 3h⁻¹).

According to the present invention, in order to more effectively remove light impurity components and heavy impurity components in isopropanol, in some embodiments, the de-light separation is carried out in a plate distillation tower or a packed distillation tower to separate the light impurity components, and the conditions for the de-light separation include: an operating reflux ratio of 2-20 (e.g., 2, 4, 5, 8, 10, 12, 15 or 20), preferably 5-15.

According to the present invention, in some embodiments, the conditions for the de-light separation include: an operating pressure of - 0.5~10 bar (for example, -0.5 bar, 0.25 bar, 1 bar, 1.5 bar, 2.0 bar, 5.0 bar, 8.0 bar or 10 bar), preferably 0.25-1.0 bar.

According to the present invention, in some embodiments, the conditions for the de-light separation include: a bottom operating temperature of 40-95°C (e.g., 40°C, 50°C, 60°C, 75°C, 85°C or 95°C), preferably 50-85°C.

According to the present invention, in some embodiments, the de-heavy separation is carried out in a plate distillation tower or a packed distillation tower to separate the heavy impurity components, and the conditions for the de-heavy separation include: an operating reflux ratio of 1-10 (e.g., 1, 2, 4, 6, 8 or 10), preferably 2-8.

According to the present invention, in some embodiments, the conditions for the de-heavy separation include: an operating pressure of-0.5 ~10 bar (e.g., -0.5 bar, 0.5 bar, 1.0 bar, 2.0 bar, 4.0 bar, 6.0 bar or 10 bar), preferably 0.5-1 bar.

According to the present invention, in some embodiments, the conditions for the de-heavy separation include: a tower top operating temperature of 50-90°C (e.g., 50°C, 60°C, 65°C, 75°C, 85°C or 90°C), preferably 65-85°C.

According to the present invention, "at least one isopropanol adsorption-filtration" means that the adsorption-filtration operation can be performed once or repeatedly for multiple times as needed.

According to the present invention, in order to remove anions and cations in isopropanol, in some embodiments, in isopropanol adsorption-filtration, the adsorption material used includes one or more of anion exchange resin and cation exchange resin.

According to the present invention, those skilled in the art may use anion exchange resin or cation exchange resin alone as the adsorption material as needed, or may select a mixed resin of anion exchange resin and cation exchange resin as the adsorption material as needed, which will not be elaborated in detail herein.

According to the present invention, in order to remove particles in isopropanol, in some embodiments, in isopropanol adsorption-filtration, the filter material used includes one or more of a polymer membrane, a ceramic membrane and a metal membrane.

By adopting the foregoing embodiments, it is possible to avoid the influence on the purity of isopropanol by the leaching of metal ions and particles that may be present in the material of the components in the pre-process section.

In another aspect, the present invention provides a system for preparing electronic-grade isopropanol by hydrogenating acetone, the system comprising:
an acetone purification unit, which is used for purifying the acetone raw material to obtain acetone feed;
an acetone hydrogenation unit, which is used for hydrogenating the acetone feed to obtain a crude isopropanol product;
an isopropanol purification unit, which is used for purifying the crude isopropanol product to obtain electronic-grade isopropanol;
wherein, the acetone purification unit comprises an acetone pre-oxidation processor, an acetone dehydration device, and an acetone refining device which are connected in sequence; the acetone hydrogenation unit comprises an acetone hydrogenation reactor; and the isopropanol purification unit comprises an isopropanol dehydration device, an isopropanol refining device, and an adsorption-filtration device which are connected in sequence.

In the present invention, the acetone pre-oxidation processor can select a tubular reactor or a kettle reactor as needed. The specific structures of the tubular reactor, the kettle reactor and the acetone hydrogenation reactor are the structures commonly used in the art.

According to the present invention, the organic impurities that are difficult to be separated from isopropanol are removed in an acetone purification unit, thereby avoiding the defects of difficulty in separating from isopropanol and high energy consumption in the later stage.

According to the present invention, in some embodiments, the acetone dehydration device comprises an acetone dehydration tower.

According to the present invention, in some embodiments, the acetone refining device comprises an acetone refining tower.

According to the present invention, in some embodiments, the isopropanol dehydration device comprises an isopropanol dehydration tower.

According to the present invention, in some embodiments, the isopropanol refining device includes an isopropanol de-light tower for removing light impurity components with a lower boiling point than isopropanol and an isopropanol de-heavy tower for removing heavy impurity components with a higher boiling point than isopropanol, which are connected in sequence.

According to the present invention, in some embodiments, the isopropanol de-light tower is a plate distillation tower or a packed distillation tower, and the isopropanol de-heavy tower is a plate distillation tower or a packed distillation tower.

In the present invention, the structures of the acetone dehydration tower, the plate distillation tower and the packed distillation tower are those commonly used in the art and will not be elaborated herein.

According to the present invention, in some embodiments, the adsorption-filtration equipment comprises an adsorption-filtration equipment.

In the system according to the present invention, a raw material pump, as well as inlets and outlets for raw materials and products, etc. can be provided as needed.

The preferred embodiments of the process and system of the present invention is described below in conjunction with FIG. 1.

As shown in FIG. 1,
the acetone raw material ① is pressurized by a raw material pump and enters an acetone pre-oxidation processor R1 filled with an oxidized preoxidant to conduct pre-oxidation to obtain pre-oxidized acetone ②; the pre-oxidized acetone ② is dehydrated by an acetone dehydration tower C1 filled with a dehydrating material to obtain dehydrated acetone ③; the dehydrated acetone ③ enters an acetone refining tower T1 to obtain a refined acetone feed ④, and a heavy component ⑤ is discharged; the refined acetone feed ④ enters an acetone hydrogenation reactor R2 filled with a hydrogenation catalyst to conduct a hydrogenation reaction to obtain a crude isopropanol product ⑥; the crude isopropanol product ⑥ is dehydrated by isopropanol dehydration tower C2 filled with dehydrating material to obtain dehydrated isopropanol ⑦; the dehydrated isopropanol ⑦ enters isopropanol de-light tower T2 to conduct de-light separation to remove light impurity components ⑧ to obtain de-light isopropanol product ⑨ ; the de-light isopropanol product ⑨ passes through isopropanol de-heavy tower T3 to conduct de-heavy separation to remove heavy impurity components ⑪ to obtain a primary isopropanol product ⑩; the primary isopropanol product ⑩ passes through adsorption-filtration equipment C3 to remove anions, cations and particles to obtain electronic-grade isopropanol ⑫.

### Example

The present invention will be described in detail below by way of examples. In the following examples and comparative examples:
the water content was determined by Karl Fischer Moisture Meter;
single metal cation concentration was determined by ICP-MS;
anion concentration was determined by ion chromatography;
the number of particles (≥50 nm) was determined by an online particle size analyzer;
unless otherwise specified, various materials are commercially available.

### Ligand Preparation Example 1:

4.2 g of diethyl tartarate, 3.2 g of 2,2-dimethylpropane, 0.42 g of p-toluenesulfonic acid, and 100 g of anhydrous toluene were added in a fully dried 250 mL three-necked flask with a reflux condenser; after nitrogen replacement, the mixture was heated to reflux for 12 hours and cooled naturally to room temperature. Saturated sodium bicarbonate solution was slowly added until the organic phase was nearly neutral, and the solvent was evaporated under reduced pressure. 120 g of anhydrous methanol was added under nitrogen atmosphere, and 7.15 g of sodium borohydride was added in batches, and the mixture was stirred at room temperature for 12 hours. After dilution with water, the organic solvent was evaporated, and the residual liquid was extracted three times with dichloromethane, the organic phases were combined, anhydrous sodium sulfate was added to conduct drying; the organic solvent was evaporated after filtration, and the residue was recrystallized to obtain 2,2-dimethyl-1,3-dioxolane-4,5-dimethanol.

### Ligand Preparation Example 2:

4.2 g of diethyl tartarate, 3.2 g of 2,2-dimethoxypropane, 0.42 g of p-toluenesulfonic acid, and 100 g of anhydrous toluene were added in a fully dried 250 mL three-necked flask with a reflux condenser; after nitrogen replacement, the mixture was heated to reflux for 12 hours and cooled naturally to room temperature. Saturated sodium bicarbonate solution was slowly added until the organic phase was nearly neutral, and the solvent was evaporated under reduced pressure. The residue was transferred to a fully dried 250 mL three-necked flask, and 120 g of anhydrous tetrahydrofuran solution containing 7.3 g of methyl magnesium iodide was added. After nitrogen replacement, the mixture was stirred at room temperature for 24 hours. After the reaction was completed, the system was cooled to -10°C in an ice-salt bath, and saturated ammonium chloride solution was slowly added until the organic phase was nearly neutral. The insoluble matter was filtered with diatomaceous earth, and the filtrate was evaporated under reduced pressure to remove the solvent. The residue was purified by column chromatography to obtain 2,2-dimethyl-1,3-dioxolane-4,5-di(1-ethanol).

### Example 1

Oxidation treatment of the pre-oxidant: by taking equimolar amounts of 2,2-dimethyl-1,3-dioxolane-4,5-dimethanol obtained in ligand preparation example 1 and manganese chloride salt, a 2,2-dimethyl-1,3-dioxolane-4,5-dimethanol solution containing 2,2-dimethyl-1,3-dioxolane-4,5-dimethanol and a manganese chloride solution containing manganese chloride were added in an anhydrous and oxygen-free nitrogen atmosphere, and stirred at room temperature for 6 hours; potassium permanganate was added hereinto to conduct reaction for 3 hours, and then the solvent was evaporated to obtain an oxidized pre-oxidant;
as shown in FIG. 1, acetone raw material ① (industrial-grade acetone, purity of 98%) was pressurized by a raw material pump and introduced into an acetone pre-oxidation processor R1 filled with an oxidized preoxidant at a flow rate of 0.6 mL/min to perform pre-oxidation to obtain pre-oxidized acetone ②; the pre-oxidized acetone ② was dehydrated by an acetone dehydration tower C1 filled with a dehydrating material (4A molecular sieve) to obtain dehydrated acetone ③ ; the dehydrated acetone ③ was introduced into an acetone refining tower T1 (plate distillation tower) for refining to obtain refined acetone feed ④, and heavy components ⑤ were discharged; the refined acetone feed ④ was introduced into an acetone hydrogenation reactor R2 filled with a hydrogenation catalyst (5 wt% Cu/SiO₂ catalyst) for hydrogenation reaction to obtain a crude isopropanol product ⑥; the crude isopropanol product ⑥ was dehydrated by an isopropanol dehydration tower C2 filled with a dehydrating material (4A molecular sieve) to obtain dehydrated isopropanol ⑦; the dehydrated isopropanol ⑦ was introduced into an isopropanol de-light tower T2 (packed tower) for de-light separation to remove light impurity components ⑧ to obtain a de-light isopropanol product ⑨; the de-light isopropanol product ⑨ was passed through an isopropanol heavy-impurities removing tower T3 (packed tower) for de-heavy separation to remove heavy impurity components ⑪ to obtain primary isopropanol product ⑩; the primary isopropanol product ⑩ then was passed through an adsorption-filtration equipment C3 to remove anions, cations and particles, to obtain electronic-grade isopropanol ⑫;
the pre-oxidation conditions including: normal temperature and pressure, the addition amount of pre-oxidant being 0.03% by weight of acetone raw material;
the refining conditions of acetone including: an operating reflux ratio of 0.5, an operating pressure of 1 bar; controlling the tower top operating temperature to 56°C;
the conditions of the hydrogenation reaction including: a reaction inlet temperature of 100°C, a reaction pressure of 4 MPa, a molar ratio of hydrogen to acetone of 8:1, and an acetone space velocity of 1.0 h⁻¹;
the conditions for the de-light separation including: an operating reflux ratio of 5, an operating pressure of 0.75 bar, and controlling the tower bottom operating temperature to 75°C;
the conditions for the de-heavy separation including: an operating reflux ratio of 5, an operating pressure of 1 bar, and controlling the tower top operating temperature to 82°C;
the adsorption-filtration equipment C3 was filled with a cation exchange resin-anion exchange resin mixed resin and a polymer filtration membrane.

The purity, water content, single metal cation concentration, anion concentration, and number of particles (≥50nm) of the electronic-grade isopropanol ⑫ are shown in Table 1.

### Example 2

According to the method of Example 1 except that:
oxidation treatment of the pre-oxidant: 2,5-dimethyl-1,3-dioxane-4,6-dimethanol was obtained in similar manner to the ligand preparation example. 2,5-dimethyl-1,3-dioxane-4,6-dimethanol and cobalt acetate were weighed in equimolar amounts, and a 2,5-dimethyl-1,3-dioxane-4,6-dimethanol solution containing 2,5-dimethyl-1,3-dioxane-4,6-dimethanol and a cobalt acetate solution containing cobalt acetate were added in anhydrous, oxygen-free, nitrogen atmosphere, and stirred at room temperature for 6 hours; potassium permanganate was added hereinto to conduct reaction for 3 hours, and then the solvent was evaporated to obtain an oxidized pre-oxidant;
dehydrating material (4A molecular sieve and anhydrous potassium carbonate composite bed layer, volume ratio being 50%-50%);
acetone refining tower T1 (packed distillation tower);
hydrogenation catalyst (Ni/SiO₂ catalyst);
wherein the refining conditions of acetone included: an operating reflux ratio of 2, an operating pressure of 1.5 bar; controlling the tower top operating temperature to 64°C;
the conditions of the hydrogenation reaction included: a reaction inlet temperature of 90°C, a reaction pressure of 4 MPa, hydrogen/acetone molar ratio of 8:1, an acetone space velocity of 1.0 h⁻¹;
the conditions for the de-light separation included: an operating reflux ratio of 5, an operating pressure of 0.5 bar; and controlling the tower bottom operating temperature to 65°C;
the conditions for the de-heavy separation included: an operating reflux ratio of 5, an operating pressure of 1.5 bar; and controlling the tower top operating temperature to 90°C;
the purity, water content, single metal cation concentration, anion concentration, and number of particles (≥50 nm) of the electronic-grade isopropanol ⑫ are shown in Table 1.

### Example 3

According to the method of Example 1 except that:
oxidation treatment of the pre-oxidant: 2,2-diethyl-1,3-dioxolane-4,5-dimethanol was obtained in similar manner to the ligand preparation example. 2,2-diethyl-1,3-dioxolane-4,5-dimethanol and manganese chloride were weighed in equimolar amounts, a 2,2-diethyl-1,3-dioxolane-4,5-dimethanol solution containing 2,2-diethyl-1,3-dioxolane-4,5-dimethanol and a manganese chloride solution containing manganese chloride were added in an anhydrous, oxygen-free, nitrogen atmosphere, and stirred at room temperature for 6 hours; potassium permanganate was added hereinto to conduct reaction for 3 hours, and then the solvent was evaporated to obtain an oxidized pre-oxidant.

The conditions of the hydrogenation reaction included: a reaction inlet temperature of 70°C, a reaction pressure of 2MPaG, the molar ratio of hydrogen to acetone of 4 : 1, and an acetone space velocity of 0.8 h⁻¹;

The conditions for the de-light separation included: an operating reflux ratio of 15, an operating pressure of 1 bar; and controlling the tower bottom operating temperature to 85°C.

The conditions for the de-heavy separation included: an operating reflux ratio of 7, an operating pressure of 1 bar; and controlling the tower top operating temperature to 85°C.

The adsorption-filtration equipment C3 was filled with mixed resin and ceramic filter membrane.

The purity, water content, single metal cation concentration, anion concentration, and number of particles (≥50nm) of the electronic-grade isopropanol ⑫ are shown in Table 1.

### Example 4

According to the method of Example 2 except that:
wherein, the refining conditions of acetone included: an operating reflux ratio of 2, an operating pressure of 0.5 bar; controlling the tower top operating temperature to 47°C;
the conditions for the de-light separation included: an operating reflux ratio of 5, an operating pressure of 1.5 bar, and controlling the tower bottom operating temperature to 90°C;
the conditions for the de-heavy separation included: an operating reflux ratio of 5, an operating pressure of 0.5 bar, and controlling the tower top operating temperature to 65°C.

The purity, water content, single metal cation concentration, anion concentration, and number of particles (≥50nm) of the electronic-grade isopropanol ⑫ are shown in Table 1.

### Example 5

According to the method of Example 2 except that:
oxidation treatment of the pre-oxidant: by taking equimolar amounts of 2,2-dimethyl-1,3-dioxolane-4,5-dimethanol obtained in the ligand preparation example 1 and molybdenum chloride, a 2,2-dimethyl-1,3-dioxolane-4,5-dimethanol solution containing 2,2-dimethyl-1,3-dioxolane-4,5-dimethanol and a molybdenum chloride solution containing molybdenum chloride were added in an anhydrous, oxygen-free, nitrogen atmosphere, and stirred at room temperature for 6 hours; potassium permanganate was added hereinto to conduct reaction for 3 hours, and then the solvent was evaporated to obtain an oxidized pre-oxidant.

The purity, water content, single metal cation concentration, anion concentration, and number of particles (≥ 50 nm) of electronic-grade isopropanol ⑫ are shown in Table 1.

### Example 6

According to the method of Example 1 except that:
pretreatment of the oxidant: by taking equimolar amounts of 2,2-dimethyl-1,3-dioxolane-4,5-dimethanol obtained in the ligand preparation example 1 and cupric chloride, a 2,2-dimethyl-1,3-dioxolane-4,5-dimethanol solution containing 2,2-dimethyl-1,3-dioxolane-4,5-dimethanol and a cupric chloride solution containing cupric chloride were added in an anhydrous, oxygen-free, nitrogen atmosphere, and stirred at room temperature for 6 hours. Potassium permanganate was added hereinto to conduct reaction for 3 hours, and then the solvent was evaporated to obtain an oxidized preoxidant.

The purity, water content, single metal cation concentration, anion concentration, and number of particles (≥ 50 nm) of electronic-grade isopropanol ⑫ are shown in Table 1.

### Example 7

According to the method of Example 1 except that:
the oxidized pre-oxidant was replaced by an oxidizing metal salt, KMnO₄.

The purity, water content, single metal cation concentration, anion concentration, and number of particles (≥ 50 nm) of electronic-grade isopropanol ⑫ are shown in Table 1.

### Example 8

According to the method of Example 1 except that:
the oxidized pre-oxidant was replaced by a 10 wt% Co oxide/SiO₂ supported catalyst.

The purity, water content, single metal cation concentration, anion concentration, and number of particles (≥ 50 nm) of electronic-grade isopropanol ⑫ are shown in Table 1.

### Example 9

According to the method of Example 1 except that:
oxidation treatment of the pre-oxidant: by taking equimolar amounts of 2,2-dimethyl-1,3-dioxolane-4,5-di(1-ethanol) obtained in the ligand preparation example 2 and manganese chloride salt, a 2,2-dimethyl-1,3-dioxolane-4,5-di(1-ethanol) solution containing 2,2-dimethyl-1,3-dioxolane-4,5-di(1-ethanol) and a manganese chloride solution containing manganese chloride were added in an anhydrous, oxygen-free, nitrogen atmosphere, and stirred at room temperature for 6 hours. Potassium permanganate was added hereinto to conduct reaction for 3 hours, and then the solvent was evaporated to obtain an oxidized preoxidant.

The results are shown in Table 1.

### Example 10

According to the method of Example 1 except that:
oxidation treatment of the pre-oxidant: 2,2-dimethyl-1,3-dioxolane-4,5-dibenzyl alcohol was obtained in similar manner to the ligand preparation example, and 2,2-dimethyl-1,3-dioxolane-4,5-dibenzyl alcohol and manganese chloride salt were weighed in equimolar amounts. In anhydrous and oxygen-free, nitrogen atmosphere, a 2,2-dimethyl-1,3-dioxolane-4,5-dibenzyl alcohol solution containing 2,2-dimethyl-1,3-dioxolane-4,5-dibenzyl alcohol and a manganese chloride solution containing manganese chloride were added and stirred at room temperature for 6 hours. Potassium permanganate was added hereinto to conduct reaction for 3 hours, and then the solvent was evaporated to obtain an oxidized pre-oxidant.

The results are shown in Table 1.

### Example 11

According to the method of Example 1 except that:
oxidation treatment of the pre-oxidant: 2,2-dimethyl-1,3-dioxolane-4,5-di-p-methylbenzyl alcohol was obtained in similar manner to the ligand preparation example, and 2,2-dimethyl-1,3-dioxolane-4,5-di-p-methylbenzyl alcohol and manganese chloride salt were weighed in equimolar amounts. In anhydrous and oxygen-free, nitrogen atmosphere, a 2,2-dimethyl-1,3-dioxolane-4,5-di-p-methylbenzyl alcohol solution containing 2,2-dimethyl-1,3-dioxolane-4,5-di-p-methylbenzyl alcohol and a manganese chloride solution containing manganese chloride were added and stirred at room temperature for 6 hours. Potassium permanganate was added hereinto to conduct reaction for 3 hours, and then the solvent was evaporated to obtain an oxidized pre-oxidant.

The results are shown in Table 1.

### Comparative Example 1

According to the method of Example 1 except that:
the acetone raw material ① was not subjected to pre-oxidation treatment, that is, the acetone raw material ① is pressurized by the raw material pump and directly introduced into the acetone dehydration tower C1 filled with dehydrating material to remove water to obtain dehydrated acetone ③, and the dehydrated acetone ③ was processed according to the subsequent procedure of Example 1 until isopropanol was obtained.

The purity, water content, single metal cation concentration, anion concentration, and number of particles (≥ 50 nm) of isopropanol are shown in Table 1.

### Comparative Example 2

According to the method of Example 2 except that:
the preoxidized acetone ② was not passed through the acetone dehydration tower C1 filled with a dehydrating material (4A molecular sieve) to remove water, that is, the preoxidized acetone ② directly was introduced into the acetone refining tower T1 (plate distillation tower) for refining to obtain the refined acetone feed ④, and the acetone feed ④ was processed according to the subsequent procedure of Example 1 until isopropanol was obtained.

The purity, water content, single metal cation concentration, anion concentration, and number of particles (≥ 50 nm) of isopropanol are shown in Table 1.

**Table 1**

| No. | Purity | Water content | Single metal cation concentrations | Anion concentration | Number of particles (≥50nm) |
|---|---|---|---|---|---|
| Example 1 | 99.9997% | 10ppm | ≤10ppt | ≤5ppb | ≤10/ml |
| Example 2 | 99.9998% | 10ppm | ≤10ppt | ≤5ppb | ≤10/ml |
| Example 3 | 99.9990% | 10ppm | ≤10ppt | ≤5ppb | ≤ 10/ml |
| Example 4 | 99.9980% | 10ppm | ≤10ppt | ≤5ppb | ≤ 10/ml |
| Example 5 | 99.9997% | 10ppm | ≤10ppt | ≤5ppb | ≤ 10/ml |
| Example 6 | 99.9910% | 10ppm | ≤10ppt | ≤5ppb | ≤ 10/ml |
| Example 7 | 99.9915% | 10ppm | ≤10ppt | ≤5ppb | ≤10/ml |
| Example 8 | 99.9916% | 10ppm | ≤10ppt | ≤5ppb | ≤10/ml |
| Example 9 | 99.9999% | 10ppm | ≤10ppt | ≤5ppb | ≤10/ml |
| Example 10 | 99.9995% | 10ppm | ≤10ppt | ≤5ppb | ≤10/ml |
| Example 11 | 99.9996% | 10ppm | ≤10ppt | ≤5ppb | ≤10/ml |
| Comparative Example 1 | 99.9880% | 15ppm | ≤10ppt | ≤5ppb | ≤ 10/ml |
| Comparative Example 2 | 99.9980% | 200ppm | ≤10ppt | ≤5ppb | ≤ 10/ml |

From the results in Table 1 it can be seen that Examples 1-10 use the process of introducing the acetone raw material into the acetone pre-oxidation processor to pre-oxidize the impurities to obtain pre-oxidized acetone in the present invention, combined with a dehydration of acetone, an acetone refining, an isopropanol dehydration, an isopropanol refining and at least one isopropanol adsorption-filtration, the overall process of Examples 1-10 is simple, has high process added value, and the organic impurities content in the obtained isopropanol product can be as low as less than 5ppm, and the anions and cations, particle content, water content, etc. can meet the requirements of high-end wafer manufacturing (line width≤90nm).

### Example 12: (Treatment of acetone containing 35 ppm of phenol)

Oxidation treatment of the pre-oxidant: by taking equimolar amounts of 2,2-dimethyl-1,3-dioxolane-4,5-dimethanol obtained in the ligand preparation example and manganese chloride salt, a 2,2-dimethyl-1,3-dioxolane-4,5-dimethanol solution containing 2,2-dimethyl-1,3-dioxolane-4,5-dimethanol and a manganese chloride solution containing manganese chloride were added in anhydrous and oxygen-free nitrogen atmosphere, and stirred at room temperature for 6 hours; potassium permanganate was added hereinto to conduct reaction for 3 hours, and then the solvent was evaporated to obtain an oxidized pre-oxidant.

As shown in FIG. 1, acetone raw material ① (industrial-grade acetone, purity of 98 %) is pressurized by a raw material pump and then introduced into an acetone pre-oxidation processor R1 filled with an oxidized pre-oxidant at a flow rate of 0.6 mL/min for pre-oxidation to obtain pre-oxidized acetone ②, wherein the phenol content was reduced to < 1 ppm.

### Example 13: (Treatment of methyl ethyl ketone containing 45 ppm acetaldehyde)

Oxidation treatment of the pre-oxidant: by taking equimolar amounts of 2,2-dimethyl-1,3-dioxolane-4,5-dimethylmethanol obtained in the ligand preparation example and manganese chloride salt, a 2,2-dimethyl-1,3-dioxolane-4,5-dimethylmethanol solution containing 2,2-dimethyl-1,3-dioxolane-4,5-dimethylmethanol and a manganese chloride solution containing manganese chloride were added in anhydrous, oxygen-free, nitrogen atmosphere, and stirred at room temperature for 6 hours; potassium permanganate was added hereinto to conduct reaction for 3 hours, and then the solvent was evaporated to obtain an oxidized pre-oxidant.

As shown in FIG. 1, methyl ethyl ketone raw material ① (industrial-grade methyl ethyl ketone, purity of 98 %) was pressurized by a raw material pump and introduced into an acetone pre-oxidation processor R1 filled with an oxidized pre-oxidant at a flow rate of 0.6 mL/min for pre-oxidation to obtain pre-oxidized acetone ②, wherein the acetaldehyde content was reduced to < 1 ppm.

### Example 14: (Treatment of toluene containing 20 ppm aniline)

Oxidation treatment of the pre-oxidant: by taking equimolar amounts of 2,2-dimethyl-1,3-dioxolane-4,5-dimethanol obtained in the ligand preparation example and manganese chloride salt, a 2,2-dimethyl-1,3-dioxolane-4,5-dimethanol solution containing 2,2-dimethyl-1,3-dioxolane-4,5-dimethanol and a manganese chloride solution containing manganese chloride were added in anhydrous and oxygen-free nitrogen atmosphere, and stirred at room temperature for 6 hours; potassium permanganate was added hereinto to conduct reaction for 3 hours, and then the solvent was evaporated to obtain an oxidized pre-oxidant.

As shown in FIG. 1, toluene raw material ① (industrial-grade toluene, purity of 98 %) was pressurized by a raw material pump and introduced into the acetone pre-oxidation processor R1 filled with oxidized pre-oxidant at a flow rate of 0.6 mL/min for pre-oxidation to obtain pre-oxidized acetone ②; wherein the phenol content was reduced to 1 ppm.

The preferred embodiments of the present invention are described in detail above, but the present invention is not limited thereto. Within the technical concept of the present invention, the technical solution of the present invention can be subjected to a variety of simple modifications, including the combinations of various technical features in any other suitable manner, and these simple modifications and combinations should also be regarded as the contents disclosed by the present invention and belong to the protection scope of the present invention.

## Claims

1. A metal oxygen-containing complex, comprising a transition metal element as central atom and an oxygen-containing heterocyclic organic compound containing a substituent as ligand;
wherein the ligand is a compound having a structure shown in formula (I), 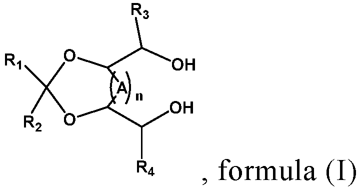
in formula (I), n is an integer of 0-5, A is -CH(R₅)-;
R₁ and R₂ at each occurrence are each independently selected from C1-C8 alkyl, and R₃, R₄ and R₅ at each occurrence are each independently selected from H, C1-C8 alkyl, phenyl C1-C8 alkyl and C1-C8 alkylphenyl.

2. The metal oxygen-containing complex according to claim 1, wherein R₁ and R₂ at each occurrence are each independently selected from C1-C5 alkyl, preferably each independently selected from C1-C4 alkyl; R₃, R₄ and R₅ at each occurrence are each independently selected from H, C1-C5 alkyl, phenyl C1-C5 alkyl and phenyl substituted with C1-C5 alkyl, preferably each independently selected from H, C1-C4 alkyl, benzyl and phenyl substituted with C1-C3 alkyl.

3. The metal oxygen-containing complex according to claim 1 or 2, wherein the transition metal element is selected from one or more of chromium, molybdenum, tungsten, iron, cobalt, nickel, ruthenium, rhodium, palladium, platinum, manganese, technetium and rhenium.

4. The metal oxygen-containing complex according to any one of the preceding claims, wherein:
in formula (I), n is an integer from 0 to 3;
R₁ and R₂ are each independently selected from H, C1-C3 alkyl, R₃ and R₄ are each independently selected from H, C1-C3 alkyl, benzyl and methyl-substituted phenyl; and
R₅ is H or C1-C3 alkyl;
preferably, in formula (I), n is 0 or 1; and
R₁ and R₂ are each independently selected from C1-C3 alkyl, R₃ and R₄ are each independently selected from H, C1-C3 alkyl, benzyl and methyl-substituted phenyl, and R₅ is H.

5. The metal oxygen-containing complex according to any one of the preceding claims, wherein the metal oxygen-containing complex is prepared by the following process: adding a ligand solution and a metal salt solution according to equimolar amounts of ligand and transition metal in anhydrous, oxygen-free, nitrogen atmosphere, stirring the mixture at room temperature for 1-10 hours to conduct a complexation reaction, and optionally evaporating the solvent after the complexation reaction.

6. A process for treating a crude material selected from ketones crude material and monocyclic aromatic hydrocarbons crude material, wherein it comprises: the metal oxygen-containing complex according to any one of the preceding claims is used as a pre-oxidant to be in contact with the crude material, thereby at least part of the impurities contained in the crude material is pre-oxidized, wherein the impurities include reducing impurities.

7. The process according to claim 6, wherein the reducing impurities are selected from alcohols, aldehydes, ketones, esters, ethers and olefins; preferably, the reducing impurities are selected from saturated or unsaturated C1-C4 primary or secondary alcohols, C1-C4 aldehydes, C1-C4 alkyl formates, C2-C4 olefins, aniline optionally substituted by C1-C4 alkyl and phenol optionally substituted by C1-C4 alkyl.

8. The process according to claim 6 or 7, wherein the amount of the pre-oxidant added is 0.01-0.1% by weight of the crude material.

9. The process according to any one of claims 6 to 8, wherein the pre-oxidant is oxidized by using an oxidant before preforming the pre-oxidation;
preferably, the oxidant is selected from one or more of oxygen, hydrogen peroxide, cumene peroxide, peracetic acid, perchlorate, permanganate and dichromate.

10. The process according to any one of claims 6 to 9, wherein the ketones are ketones containing C1-C8 alkyl, C6-C8 aryl, C2-C8 ester group or halogen, and the monocyclic aromatic hydrocarbons are monocyclic aromatic hydrocarbons containing C1-C8 alkyl, C2-C8 ester group or halogen; preferably, the crude material is acetone raw material.

11. The process according to claim 10, wherein the crude material is acetone raw material, and the pre-oxidation of the acetone raw material is performed as step (1) in a pre-oxidation processor to obtain a pre-oxidized acetone raw material;
and the process also includes the following steps:
(2) introducing an acetone feed into an acetone hydrogenation reactor to conduct hydrogenation reaction to obtain a crude isopropanol product; wherein the acetone feed is the preoxidized acetone raw material obtained from step (1), or the preoxidized acetone raw material is optionally subjected to intermediate treatment (1-2) to obtain a refined acetone raw material.

12. The process according to claim 10, wherein the intermediate treatment (1-2) is performed between the steps (1) and (2), and the intermediate treatment (1-2) comprises: introducing the pre-oxidized acetone raw material into an acetone dehydration device and an acetone refining device in sequence to perform acetone dehydration and acetone refining to obtain refined acetone.

13. The process according to claim 10 or 11, further comprising the step (3) of post-treating the crude isopropanol product: sequentially passing the crude isopropanol product through an isopropanol dehydration device, an isopropanol refining device and an adsorption-filtration device to perform isopropanol dehydration, isopropanol refining and at least one isopropanol adsorption-filtration, to obtain electronic-grade isopropanol.

14. The process according to any one of claims 10 to 13, wherein:
in step (1), the pre-oxidation conditions include:
the amount of the pre-oxidant added is 0.01-0.1% by weight of the acetone raw material; and
at normal temperature and normal pressure.

15. The process according to any one of claims 10 to 14, wherein: in step (1), the acetone raw material includes one or more of industrial-grade acetone, analytical-grade acetone and electronic-grade acetone.
